# EUROPÄISCHE PATENTSCHRIFT

(11) **EP 1 518 850 B1**
(45) Veröffentlichungstag und Bekanntmachung des Hinweises auf die Patenterteilung: **20.06.2007**
(21) Anmeldenummer: 04020370.5
(22) Anmeldetag: 27.08.2004
(51) Int. Cl.: C07C 29/10, C07C 29/151, C07C 31/12

(54) **Enantiomerenangereichertes 2-Butanol**
Enantiomer-enriched 2-butanol
2-butanole enantiomèriquement enrichi

(30) Priorität: 08.09.2003 DE 10341270
(43) Veröffentlichungstag der Anmeldung: 30.03.2005
(73) Patentinhaber: LANXESS Deutschland GmbH, 51369 Leverkusen (DE)
(72) Erfinder: Schlummer, Björn, Dr., 51377 Leverkusen (DE); Stürmann, Martin, Dr., 51375 Leverkusen (DE)

(56) Entgegenhaltungen:
- EP-A- 0 345 243
- SAJIKI, HIRONAO ET AL: "Pd/C(en)-catalyzed regioselective hydrogenolysis of terminal epoxides to secondary alcohols" 1999, CHEMICAL COMMUNICATIONS (CAMBRIDGE) , (11), 1041-1042 CODEN: CHCOFS; ISSN: 1359-7345 , XP001204954 * das ganze Dokument *
- J.COKE AND R.SHUE: "Nucleophilic Ring Opening of Optically Pure (R)-(+)-1,2-Epoxybutane. Synthesis of New (R)-2-Butanol Derivatives" J.ORG.CHEM, Bd. 38, Nr. 12, 1973, Seiten 2210-2211, XP001204975

## Beschreibung

Die vorliegende Erfindung betrifft ein Verfahren zur Herstellung von enantiomerenangereichertem 2-Butanol sowie seine Verwendung.

Chirale, enantiomerenreine Alkohole sind wichtige Bausteine in der Synthese von hochveredelten Produkten wie Feinchemikalien oder pharmazeutischen Zwischenprodukten. Die Synthese von chiralen, enantiomerenreinen Alkoholen ist prinzipiell über eine Vielzahl von chemischen Transformationen möglich.

So ist beispielsweise aus T. Ohkuma und R. Noyori, Hydrogenation of Carbonyl groups in Comprehensive Asymmetric Catalysis, 1999, Eds.: E. N. Jacobsen, A. Pfaltz, H. Yamamoto, Springer Verlag, Berlin, Heidelberg, New York, S. 199-247 die enantioselektive Hydrierung von Ketonen mit chiralen Rutheniumkatalysatoren, beschrieben.

Weiterhin ist aus S. Itsuno, Hydroboration of Carbonyl groups in Comprehensive Asymmetric Catalysis, 1999, Eds.: E. N. Jacobsen, A. Pfaltz, H. Yamamoto, Springer Verlag, Berlin, Heidelberg, New York, S. 290-315 bekannt, Ketone mit chiralen Boranreagenzien asymmetrisch zu reduzieren.

Befriedigende Enantiomerenüberschüsse werden allerdings bei beiden Verfahren nur dann erhalten, wenn die zwei Substituenten an der Carbonylgruppe einen stark unterschiedlichen sterischen Anspruch aufweisen.

Alternativ dazu kommt auch die Trennung von racemischen 2-Alkoholen in Frage. Allerdings verlaufen die bekannten chemischen Methoden immer über kovalente Verknüpfungen mit einem chiralen Auxiliar, das nach erfolgter Trennung wieder aus dem Molekül entfernt werden muss und somit den Gesamtprozess unwirtschaftlich machen. Überdies zeigen solche Trennmethoden bei nur wenig unterschiedlicher Sterik der Substituenten, wie dies insbesondere bei 2-Butanol der Fall ist, nur geringe Enantioselektivitäten.

Racemische 2-Alkanole können beispielsweise durch regioselektive Hydrierung von terminalen Epoxiden erhalten werden (H. Sajiki, K. Hattori und K. Hirota, Chem. Comm. 1999, 1041-1042).

Es bestand das Bedürfnis, ein Verfahren bereitzustellen, über das enantiomerenangereichertes 2-Butanol in einfacher und effizienter Weise zugänglich ist.

Es wurde nun ein Verfahren zur Herstellung von enantiomerenangereichertem 2-Butanol gefunden, das dadurch gekennzeichnet ist, dass enantiomerenangereichertes 1,2-Epoxybutan in Gegenwart von Palladium und/oder Palladiumverbindungen und einem vicinalen Diamin mit Wasserstoff reduziert wird.

Der Begriff "enantiomerenangereichertes 2-Butanol" steht im Sinne dieser Erfindung für Mischungen der jeweiligen (R)- und (S)-Enantiomere, in denen ein Enantiomer in einem Enantiomerenüberschuss, im Folgenden auch ee ("enantiomeric excess") genannt, im Vergleich zum anderen Enantiomer vorliegt. Bevorzugt beträgt dieser ee-Wert 90 bis 100%, besonders bevorzugt 98 bis 100 % und ganz besonders bevorzugt 99 bis 100 %, wobei für letzteren Bereich auch der Begriff "enantiomerenrein" verwendet wird.

Bevorzugt wird über das erfmdungsgemäße Verfahren (S)-2-Butanol aus (R)-1,2-Epoxybutan hergestellt.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Das erfindungsgemäße Verfahren wird in Gegenwart von Palladium und/oder Palladiumverbindungen durchgeführt. Dafür eignet sich beispielsweise elementares Palladium wie Palladium-Mohr oder auf einen Träger aufgebrachtes Palladium. Als Träger geeignet sind beispielsweise Aktivkohle, Graphit, Kieselgur, Kieselgel, Spinelle, Aluminiumoxide, Calciumcarbonat, Magnesiumoxid, Bariumsulfat oder auch organische Trägermaterialien.

Weiterhin eignen sich in organischen Lösungsmitteln weitestgehend unlösliche Palladiumverbindungen wie zum Beispiel solche die durch Umsetzung von Palladium(0)- oder vorzugsweise Palladium(II)-Verbindungen mit Hydroxid erhältlich sind.

Bevorzugte Palladium (II)-Verbindungen sind dabei Verbindungen der Formeln (Ia) und (Ib)

M₂[Pd(Hal)₄] (Ia),

Pd(Hal)₂ (Ib),

in denen
- M: für Lithium, Natrium, Kalium, Ammonium oder organisches Ammonium steht und
- Hal: für Chlorid, Bromid oder Iodid steht und

Bevorzugt werden als Hydroxide Alkali- oder Erdalkalimetallhydroxide, bevorzugt Alkalimetallhydroxide wie Natrium- oder Kaliumhydroxid eingesetzt.

Die Palladiumverbindungen können als solche oder auf einem Träger aufgebracht verwendet werden. Als Träger eignen sich die oben genannten Materialien in gleicher Weise. Gegebenenfalls kann vor dem Einsatz in das erfindungsgemäße Verfahren noch eine Reduktion z.B. mit Hydrazin erfolgen.

Bevorzugt werden als Katalysatoren eingesetzt: auf einen Träger aufgebrachtes Palladium oder auf Träger aufgebrachte Palladiumverbindungen, die durch Umsetzung von Verbindungen der Formel (Ia) und/oder (Ib) mit Hydroxid gewonnen werden. Bevorzugte Träger sind pulverförmige Aktivkohlen.

Der Palladium-Gehalt von Katalysatoren auf Trägern ist unkritisch und kann innerhalb eines großen Bereichs variiert werden. Im Allgemeinen verwendet man Katalysatoren auf Trägern, deren Gehalt bezogen auf Palladium zwischen 0,1 und 20 Gew.-%, vorzugsweise zwischen 0,5 und 15 Gew.-% liegt.

Auch die Menge, in der das Palladium und/oder die Palladiumverbindung eingesetzt wird, kann in einem größeren Bereich variiert werden. Im Allgemeinen wählt man die Menge an Palladium und/oder Palladiumverbindung so, dass das molare Verhältnis von Palladium zu eingesetztem 1,2-Epoxybutan zwischen 1:1 und 1:100000, bevorzugt zwischen 1:5 und 1:1000 und besonders bevorzugt zwischen 1:10 und 1:10000 liegt.

Das erfindungsgemäße Verfahren wird weiterhin in Gegenwart von vicinalem Diamin durchgeführt. Bevorzugte vicinale Diamine sind solche der Formel (II)

H₂N-CR¹-CR²-NH₂ (II)

in der
- R¹ und R²: jeweils unabhängig voneinander für Wasserstoff, Alkyl oder Aryl steht oder
- CR¹-CR²: als Ganzes für einen 5 bis 7-gliedrigen Cycloylkylrest steht.

**Alkyl** steht dabei beispielsweise und bevorzugt für unverzweigte, verzweigte, cyclische oder acyclische C₁-C₁₂-Alkylreste, die entweder nicht oder zumindest teilweise durch Fluor, Chlor, oder unsubstituiertes oder substituiertes Aryl substituiert sein können. Besonders bevorzugt steht Alkyl für verzweigte, cyclische oder acyclische C₁-C₁₂-Alkylreste, die nicht weiter substituiert sind.

**Aryl** steht dabei beispielsweise für carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe freies oder geschütztes Hydroxy, Iod, Brom, Chlor, Fluor, Cyano, C₁-C₁₂-Alkyl wie zum Beispiel Methyl, Ethyl, n-Propyl, iso-Propyl, n-Butyl, tert.-Butyl, Cyclohexyl, n-Hexyl, n-Octyl oder iso-Octyl, C₆-C₁₂-Aryl, wie zum Beispiel Phenyl, oder C₁-C₆-Alkoxy.

Vorzugsweise steht Aryl für carbocyclische aromatische Reste mit 6 bis 18 die nicht weiter substituiert sind.
- R¹ und R²: stehen bevorzugt jeweils identisch für Wasserstoff, Methyl oder Phenyl oder
- CR¹-CR²: als Ganzes für Cyclohexandiyl oder Cyclopentandiyl.

Ein besonders bevorzugtes vicinales Diamin ist Ethylendiamin.

Die Menge an vicinalem Diamin kann beispielsweise 0,05 bis 100 mol pro mol Palldium oder Palladiumverbindung betragen, bevorzugt 0,1 bis 20 mol und ganz besonders bevorzugt 0,1 bis 2,5 mol.

Größere Mengen an vicinalem Diamin sind möglich aber unwirtschaftlich.

In einer Ausführungsform des erfmdungsgemäßen Verfahrens wird das Palladium auf einem Träger vor Einbringung in die Reaktion mit der angegebenen Menge an vicinalem Diamin für mindestens 30 min vorzugsweise 30 min bis 12 Stunden versetzt und der dabei entstehende modifizierte Katalysator vor dem Einsatz in das erfindungsgemäße Verfahren getrocknet.

In einer alternativen Ausführungsform des erfindungsgemäßen Verfahrens werden die Verbindungen der Formel (Ia) oder (Ib) mit Hydroxid umgesetzt, das resultierende Produkt gegebenenfalls nach Waschen und gegebenenfalls nach Reduktion z.B. mit Hydrazin zunächst mit der angegebenen Menge an vicinalem Diamin und dann mit dem Träger für mindestens 30 min vorzugsweise 30 min bis 12 Stunden versetzt und der dabei entstehende modifizierte Katalysator vor dem Einsatz in das erfindungsgemäße Verfahren getrocknet.

In einer weiteren alternativen Ausführungsform des erfindungsgemäßen Verfahrens werden die Verbindungen der Formel (Ia) oder (Ib) mit Hydroxid in Gegenwart des Trägers umgesetzt und das resultierende Produkt gegebenenfalls nach Waschen und gegebenenfalls nach Reduktion z.B. mit Hydrazin mit der angegebenen Menge an vicinalem Diamin für mindestens 30 min vorzugsweise 30 min bis 12 Stunden versetzt und anschließend der dabei entstehende modifizierte Katalysator vor dem Einsatz in das erfindungsgemäße Verfahren getrocknet.

In einer besonders bevorzugten Ausführungsform des erfindungsgemäßen Verfahrens werden die Verbindungen der Formel (Ia) oder (Ib) mit Hydroxid in Gegenwart des Trägers umgesetzt und das resultierende Produkt gegebenenfalls nach Waschen und gegebenenfalls nach Reduktion z.B. mit Hydrazin oder alternativ dazu auf einen Träger aufgebrachtes Palladium direkt für das erfindungsgemäße Verfahren eingesetzt und vicinales Diamin zur Reaktionsmischung dazugegeben.

Die Reaktion kann vorzugsweise ohne gegebenenfalls jedoch auch in einem organischen Lösungsmittel durchgeführt werden.

Als organische Lösungsmittel zur Durchführung des erfindungsgemäßen Verfahrens eignen sich insbesondere organische Lösungsmittel. Geeignete organische Lösungsmittel sind beispielsweise aliphatische, alicyclische oder aromatische, gegebenenfalls halogenierte Kohlenwasserstoffe, wie beispielsweise verschiedene Benzine, Benzol, Toluol, Xylol, Chlorbenzol, Dichlorbenzol, verschiedene Petrolether, Hexan, Cyclohexan, Dichlormethan, Chloroform, Tetrachlorkohlenstoff; Alkohole, wie Methanol, Ethanol, n- oder i-Propanol oder Gemische solcher organischen Lösungsmittel.

Die Reaktionstemperatur kann beispielsweise von 0 bis 150°C, bevorzugt 10 bis 100°C und besonders bevorzugt von 20 bis 50°C betragen.

Der Wasserstoffpartialdruck kann beispielsweise 0,8 bis 200 bar, bevorzugt 5 bis 100 bar und besonders bevorzugt von 10 bis 50 bar betragen.

In einer bevorzugten Ausführungsform wird der Katalysator zurückgewonnen und erneut für das erfindungsgemäße Verfahren eingesetzt. Die Rückgewinnung kann beispielsweise durch Sedimentieren und Dekantieren oder Filtration erfolgen. Ein merklicher Aktivitätsverlust wird nicht beobachtet.

Das erfindungsgemäß erhältliche enantiomerenangereicherte 2-Butanol eignet sich insbesondere zur Herstellung von Feinchemikalien, pharmazeutischen Wirkstoffen oder Zwischenprodukten davon. Bevorzugt ist dabei (S)-2-Butanol.

Der Vorteil vorliegender Erfindung ist, dass in unerwarteter Weise durch das erfindungsgemäße Verfahren eine hochregioselektive Epoxidöffnung bereitgestellt werden kann, die ohne geringste Anzeichen von Racemisierung verläuft und somit einen eleganten Zugang zu enantiomerenangereichertem 2-Butanol erlaubt.

### Beispiele

In allen nachfolgenden Beispielen wird die optische Reinheit des (R)- bzw. (S)-2-Butanols durch Gaschromatographie an einem chiralen Säulenmaterial bestimmt und über den ee-Wert angegeben. Der Begriff "Ratio" beschreibt das Verhältnis zwischen 2-Butanol und 1-Butanol, den beiden möglichen Produkten aus der Hydrierung von 1,2-Epoxybutan.

### Beispiel 1

### Präparation von Katalysator A

50,0 g (0,047 mol) Palladium auf Kohle (10 % Gew-%) werden mit 93 ml Lösung enthaltend 5,65 g (0,094 mol) 1,2-Diaminoethan und 87 ml Wasser angeteigt und 2h bei 50°C und einem Druck von 200 mbar getrocknet. Es werden 53g des Katalysators A erhalten.

### Beispiel 2

### Präparation von Katalysator B

33,3 g wässrige Na₂PdCl₄-Lsg. (0,047 mol) werden mit Wasser auf 300 ml aufgefüllt. Danach werden 150 ml 1 N Natronlauge zugesetzt. Der ausgefallene Feststoff wird mit 10 Litern Wasser dekantierend gewaschen und der Feststoff abgesaugt. Der Feststoff wird unter Rühren mit 5,65g (0,094 mol) 1,2-Diaminoethan versetzt und die sich bildende Lösung mit Wasser auf 84 ml aufgefüllt. Nach 2h werden 45g Aktivkohle zugesetzt und die Suspension bei 50°C und einem Druck von 200 mbar getrocknet. Es werden 59g Katalysator B erhalten.

### Beispiel 3

### Präparation von Katalysator C

30,0 g Na₂PdCl₄-Lsg. (0,042 mol) werden mit Wasser auf 300 ml aufgefüllt. Danach werden 135 ml 1 N Natronlauge zugesetzt. Der ausgefallene Feststoff wird mit 10 Litern Wasser dekantierend gewaschen und der Feststoff abgesaugt. Der Feststoff wird unter Rühren mit 5,09g (0,085 mol) 1,2-Diaminoethan versetzt und die sich bildende Lösung mit Wasser auf 120 ml aufgefüllt. Nach 2h wird der getrocknete Katalysator mit dieser Lösung angeteigt und bei 50°C und einem Druck von 200 mbar getrocknet. Es werden 61,1g des Katalysators C erhalten.

### Beispiel 4

### Präparation von Katalysator D

45,0g Aktivkohle werden in 300 ml Wasser suspendiert. Die Aufschlämmung wird mit 50 ml Lösung enthaltend 3,33g Na₂PdCl₄-Lösung (0,5g Palladium) und 47g Wasser versetzt. Nach 1h wird mit 10%iger Natronlauge ein pH-Wert von 8-9 eingestellt. Nach 2h wird der Katalysator auf der Nutsche chloridfrei und neutral gewaschen. Der Feststoff wird dann bei 110°C und einem Druck von 200 mbar getrocknet.
Der Katalysator wird mit 120 ml Lösung enthaltend 5,65g (0,094 mol) 1,2-Diaminoethan und 115 ml Wasser angeteigt und 2h bei 50°C und einem Druck von 200 mbar getrocknet. Es werden 59,5g des Katalysators D erhalten.

### Beispiel 5

### Präparation von Katalysator E

45,0g Aktivkohle werden in 300 ml Wasser suspendiert. Die Aufschlämmung wird mit 50 ml Lösung enthaltend 33,3g Na₂PdCl₄-Lösung (5,0g Palladium) und 17g Wasser versetzt. Nach 1h wird mit 10%iger Natronlauge ein pH-Wert von 8-9 eingestellt. Nach 2h wird die Suspension mit 33ml Hydrazinhydrat reduziert. Der Katalysator wird auf der Nutsche chloridfrei und neutral gewaschen. Der Feststoff wird dann bei 110°C und einem Druck von 200 mbar getrocknet.

Der Katalysator wird mit 120 ml Lösung enthaltend 5,65g (0,094 mol) 1,2-Diaminoethan und 115 ml Wasser angeteigt und 2h bei 50°C und einem Druck von 200 mbar getrocknet. Es werden 57,3g des Katalysators E erhalten.

### Beispiele 6-12

### Allgemeine Arbeitsvorschrift A zur Hydrierung von (R)-1,2-Epoxybutan

1 Äquivalent (R)-1,2-Epoxybutan wird in der angegebenen Menge eines organischen Lösungsmittels gelöst und in einen geeigneten Autoklaven gegeben. Es werden 10 Gew-% (relativ zum Substrat) des verwendeten Palladium-Kontaktes zugesetzt. Der Autoklav wird verschlossen und der gewünschte Wasserstoffdruck eingestellt. Das Reaktionsgemisch wird bei der angegebenen Temperatur hydriert.

Nach Ende der Reaktion wird der Autoklav entspannt und der Katalysator über Celite abfiltriert. Die chemische und optische Reinheit des so erhaltenen Filtrates, sowie der Umsatz werden mittels Gaschromatographie bestimmt.

### Beispiel 6

### Hydrierung mit Katalysator A

| **(R)-1,2-Epoxybutan [g]/(mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 100:0 | 100 | 20,4 |
| 3,35 (46,5) | 0,335 | - | - | 5 | 25 | 24 | 100:0 | 100 | 19,6 |
| 3,35 (46,5) | 0,335 | - | - | 10 | 25 | 24 | 100:0 | 100 | 33,4 |
| 45,0 (624) | 4,50 | - | - | 50 | 25 | 24 | 99:1 | 100 | 99,2 |

### Beispiel 7

### Hydrierung mit Katalysator B

| **(R)-1,2-Epoxybutan [g]/[mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 100:0 | 100 | 3,7 |
| 3,35 (46,5) | 0,335 | - | - | 5 | 25 | 24 | 100:0 | 100 | 14,1 |
| 3,35 (46,5) | 0,335 | - | - | 10 | 25 | 24 | 100:0 | 100 | 13,3 |

### Beispiel 8

### Hydrierung mit Katalysator C

| **(R)-1,2-Epoxybutan [g]/[mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 100:0 | 100 | 13,4 |
| 3,35 (46,5) | 0,335 | - | - | 5 | 25 | 24 | 100:0 | 100 | 13,0 |

### Beispiel 9

### Hydrierung mit Katalysator D

| **(R)-1,2- Epoxybutan [g]/[mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 99:1 | 100 | 42,0 |
| 3,35 (46,5) | 0,335 | - | - | 5 | 25 | 24 | 99:1 | 100 | 72,1 |
| 3,35 (46,5) | 0,335 | - | - | 5 | 25 | 24 | 99:1 | 100 | 85,7 |

### Beispiel 10

### Hydrierung mit Katalysator E

| **(R)-1,2- Epoxybutan [g]/[mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 100:0 | 100 | 6,6 |
| 3,35 (46,5) | 0,335 | - | - | 5 | 25 | 24 | 100:0 | 100 | 1,7 |

### Beispiel 11

### Hydrierung mit Palladium auf Kohle (10 Gew.-%).

| **(R)-1,2- Epoxybutan [g]/[mmol]** | **Katalysator [g]** | **Lösungsmittel** | **ml** | **P [bar]** | **T [°C]** | **t [h]** | **Ratio** | **ee (S) [%]** | **Umsatz [%]** |
|---|---|---|---|---|---|---|---|---|---|
| 0,29 (4,0) | 0,029 | MeOH | 8 | 5 | 25 | 24 | 88:12 | 100 | 92,9 |

### Beispiel 12

### Hydrierung mit Palladium auf Kohle (10 Gew.-%) unter Zusatz von 1,2-Diaminoethan bei Hydrierung ohne Lösungsmittel:

| **(R)-1,2- Epoxybutan [g]/[mmol]** | **Katalysator [g]** | **1,2- Diaminoethan [g]** | **P [bar]** | **T [°C]** | **t [h]** | **Ratio** | **ee (S) [%]** | **Umsatz [%]** |
|---|---|---|---|---|---|---|---|---|
| 3,35 (46,5) | 0,335 | - | 5 | 25 | 24 | 89:11 | 100 | 100,0 |
| 3,35 (46,5) | 0,335 | 0,020 | 10 | 25 | 24 | 99:1 | 100 | 95,8 |
| 3,35 (46,5) | 0,335 | 0,020 | 10 | 50 | 24 | 98:2 | 100 | 99,2 |
| 3,35 (46,5) | 0,335 | 0,040 | 10 | 50 | 24 | 98:2 | 100 | 98,4 |
| 3,35 (46,5) | 0,335 | 0,010 | 10 | 25 | 24 | 98:2 | 100 | 97,8 |
| 3,35 (46,5) | 0,335 | 0,010 | 10 | 50 | 24 | 97:3 | 100 | 98,6 |
| 3,35 (46,5) | 0,335 | 0,005 | 10 | 25 | 24 | 98:2 | 100 | 96,9 |

## Patentansprüche

1. Verfahren zur Herstellung von enantiomerenangereichertem 2-Butanol, **dadurch gekennzeichnet, dass** enantiomerenangereichertes 1,2-Epoxybutan in Gegenwart von Palladium und/oder Palladiumverbindungen und einem vicinalen Diamin mit Wasserstoff reduziert wird.

2. Verfahren nach Anspruch 1, **dadurch gekennzeichnet, dass** (R)-1,2-Epoxybutan zu (S)-2-Butanol reduziert wird.

3. Verfahren nach mindestens einem der Ansprüche 1 und 2, **dadurch gekennzeichnet, dass** als Palladium und/oder Palladiumverbindungen eingesetzt werden: metallisches Palladium oder auf einen Träger aufgebrachtes Palladium oder solche die durch Umsetzung von Palladium(0)- oder vorzugsweise Palladium(II)-Verbindungen mit Hydroxid erhältlich sind.

4. Verfahren nach mindestens einem der Ansprüche 1 bis 3, **dadurch gekennzeichnet, dass** das molare Verhältnis von Palladium zu eingesetztem 1,2-Epoxybutan zwischen 1:1 und 1:100000 liegt.

5. Verfahren nach mindestens einem der Ansprüche 1 bis 4, **dadurch gekennzeichnet, dass** als vicinale Diamine solche der Formel (II) eingesetzt werden,
H₂N-CR¹-CR²-NH₂ (II)
in der
R¹ und R² jeweils unabhängig voneinander für Wasserstoff, Alkyl oder Aryl steht oder
CR¹-CR² als Ganzes für einen 5 bis 7-gliedrigen Cycloylkylrest steht.

6. Verfahren nach mindestens einem der Ansprüche 1 bis 5, **dadurch gekennzeichnet, dass** es so durchgeführt wird, dass Verbindungen der Formeln (Ia) und (Ib)
M₂[Pd(Hal)₄] (Ia),
Pd(Hal)₂ (Ib)
in denen
M für Lithium, Natrium, Kalium, Ammonium oder organisches Ammonium steht und
Hal für Chlorid, Bromid oder Iodid steht mit Hydroxid in Gegenwart eines Trägers umgesetzt werden und das resultierende Produkt gegebenenfalls nach Waschen und gegebenenfalls nach Reduktion z.B. mit Hydrazin sowie vicinales Diamin direkt zur Reaktionsmischung dazugegeben werden.

7. Verfahren nach mindestens einem der Ansprüche 1 bis 6, **dadurch gekennzeichnet, dass** der Wasserstoffpartialdruck 0,8 bis 200 bar beträgt.

8. Verfahren nach mindestens einem der Ansprüche 1 bis 7, **dadurch gekennzeichnet, dass** das Palladium und/oder die Palladiumverbindung zurückgewonnen und erneut für das erfindungsgemäße Verfahren eingesetzt wird.

## Claims

1. Process for preparing enantiomerically enriched 2-butanol, **characterized in that** enantiomerically enriched 1,2-epoxybutane is reduced with hydrogen in the presence of palladium and/or palladium compounds and a vicinal diamine.

2. Process according to Claim 1, **characterized in that** (R)-1,2-epoxybutane is reduced to (S)-2-butanol.

3. Process according to at least one of Claims 1 and 2, **characterized in that** the palladium and/or palladium compounds used are: metallic palladium or palladium applied to a support or those which are obtainable by reacting palladium(0) or preferably palladium(II) compounds with hydroxide.

4. Process according to at least one of Claims 1 to 3, **characterized in that** the molar ratio of palladium to 1,2-epoxybutane used is between 1:1 and 1:100 000.

5. Process according to at least one of Claims 1 to 4, **characterized in that** the vicinal diamines used are those of the formula (II)
H₂N-CR¹-CR²-NH₂ (II)
in which
R¹ and R² are each independently hydrogen, alkyl or aryl, or
CR¹-CR² as a whole is a 5- to 7-membered cycloalkylene radical.

6. Process according to at least one of Claims 1 to 5, **characterized in that** it is carried out in such a way that compounds of the formulae (Ia) and (Ib)
M₂[Pd(Hal)₄] (Ia),
Pd(Hal)₂ (Ib)
in which
M is lithium, sodium, potassium, ammonium or organic ammonium and
Hal is chloride, bromide or iodide are reacted with hydroxide in the presence of a support and the resulting product, optionally after washing and optionally after reduction, for example with hydrazine, and also vicinal diamine are added directly to the reaction mixture.

7. Process according to at least one of Claims 1 to 6, **characterized in that** the partial hydrogen pressure is 0.8 to 200 bar.

8. Process according to at least one of Claims 1 to 7, **characterized in that** the palladium and/or the palladium compound is recovered and reused for the process according to the invention.

## Revendications

1. Procédé pour la préparation de 2-butanol enrichi en énantiomère, **caractérisé en ce qu'**on réduit du 1,2-époxybutane enrichi en énantiomère avec de l'hydrogène en présence de palladium et/ou de composés contenant du palladium et d'une diamine vicinale.

2. Procédé selon la revendication 1, **caractérisé en ce qu'**on réduit du (R)-1,2-époxybutane en (S)-2-butanol.

3. Procédé selon au moins l'une des revendications 1 et 2, **caractérisé en ce qu'**on utilise en tant que palladium et/ou composés contenant du palladium : du palladium métallique ou du palladium appliqué sur un support ou ceux qui sont obtenus par réaction de composés contenant du palladium-(0) ou de préférence du palladium-(II) avec un hydroxyde.

4. Procédé selon au moins l'une des revendications 1 à 3, **caractérisé en ce que** le rapport molaire du palladium au 1,2-époxybutane utilisé est compris entre 1:1 et 1:100 000.

5. Procédé selon au moins l'une des revendications 1 à 4, **caractérisé en ce qu'**on utilise comme diamines vicinales celles de formule (II),
H₂N-CR¹-CR²-NH₂ (II)
dans laquelle
R¹ et R² représentent chacun, indépendamment l'un un de l'autre, un atome d'hydrogène ou un groupe alkyle ou aryle, ou
CR¹-CR² représente dans son ensemble un radical cycloalkyle à 5-7 chaînons.

6. Procédé selon au moins l'une des revendications 1 à 5, **caractérisé en ce qu'**on procède en faisant réagir avec un hydroxyde, en présence d'un support, des composés de formules (Ia) et (Ib)
M₂[Pd(Hal)₄] (Ia),
Pd(Hal)₂ (Ib)
dans lesquelles
M représente le lithium, le sodium, le potassium, l'ion ammonium ou un ion ammonium organique et
Hal représente le chlore, le brome ou l'iode
et en ajoutant directement au mélange réactionnel le produit résultant, éventuellement après lavage et éventuellement après réduction, par exemple avec de l'hydrazine, ainsi qu'une diamine vicinale.

7. Procédé selon au moins l'une des revendications 1 à 6, **caractérisé en ce que** la pression partielle d'hydrogène est de 0,8 à 200 bars.

8. Procédé selon au moins l'une des revendications 1 à 7, **caractérisé en ce qu'**on récupère le palladium et/ou le composé contenant du palladium et on l'utilise à nouveau pour le procédé selon l'invention.
